# EUROPEAN PATENT APPLICATION

(11) **EP 4 060 316 A1**
(43) Date of publication of application: **21.09.2022**
(21) Application number: 20886860.4
(22) Date of filing: 28.08.2020
(51) Int. Cl.: G01N 15/12, G01N 23/2273

(54) **METHOD FOR MANUFACTURING PARTICLE ANALYSIS DEVICE, AND PARTICLE ANALYSIS DEVICE**

(30) Priority: 11.11.2019 JP 2019204178
(71) Applicant: NOK Corporation, Tokyo 105-8585 (JP)
(72) Inventor: YOSHITOMI, Takumi, Tsujido-shinmachi, Fujisawa-shi, Kanagawa 251- 0042 (JP); MUROTA, Yuki, Tsujido-shinmachi, Fujisawa-shi, Kanagawa 251- 0042 (JP); FUJISAWA, Naohiro, Tsujido-shinmachi, Fujisawa-shi, Kanagawa 251- 0042 (JP)
(74) Representative: TBK
(86) International application number: PCT/JP2020/032577
(87) International publication number: WO 2021/095325

(57) **Abstract**

Provided is a method for manufacturing a particle analyzer in which the deterioration of the measurement function is suppressed during the measurement of the particles to be measured.

The method of manufacturing a particle analyzer, the particle analyzer including a first storage chamber in which a first liquid is stored, a second storage chamber in which a second liquid containing particles to be analyzed is stored, and a flow path connecting the first storage chamber in fluid communication with the second storage chamber, which includes a surface modification step of irradiating a surface constituting the flow path with an ultraviolet ray to modify the surface of the flow path.

## Description

### Technical Field

The present invention relates to a method for manufacturing a particle analyzer and the particle analyzer. More particularly, it relates to a method for manufacturing a particle analyzer for detecting and analyzing a particle such as exosomes, etc. and the particle analyzer.

### Background Art

Conventionally, in order to detect and analyze a particle such as exosomes, pollen, viruses, bacteria, and DNA, a detection method using nanopores (see, for example, Patent Document 1) and a particle analyzer having the nanopores (see, for example, Patent Document 2) have been proposed.

The particle analyzer specifically analyses the particles in the following manner. The particle analyzer has a pore connecting two spaces, the one space stores liquid, and the other space stores liquid containing the particles to be analyzed. These spaces are given different potentials, and the particles pass through the pore by electrophoresis. When the particles pass through the pore, a change in a value of the current flowing through the liquid is measured. In this manner, the characteristics, for example, type, shape, and size, of the particles that have passed through the pore can be analyzed.

When particle detection is performed using a particle analyzer in which nanopores are formed, surface treatment of the surface constituting the nanopores or the like with a predetermined treatment solution is performed from the viewpoint that the liquids or particles are required to flow in the nanopores without clogging (see, for example, Patent Document 3).

### Citation List

### Patent Documents

[Patent Document 1] JP-A-2014-174022
[Patent Document 2] JP-B-5866652
[Patent Document 3] WO 2018/105229

### Summary of the Invention

### Problem to be Solved by the Invention

However, when the surface treatment is performed as described in Patent Document 3, there is room for further improvement in the effect due to the surface treatment. Specifically, in the case of the surface treatment with a treatment solution or the surface treatment with an oxygen plasma as described in Patent Document 3, wettability is improved, and as a result, it is possible to facilitate an operation when a solution such as a liquid containing the particles to be analyzed is introduced into the particle analyzer. However, on the other hand, the particles were clogged with the nanopores during the measurement of the particles to be measured, and the measurement function of the particles in the particle analyzer tended to deteriorate. Therefore, it has been desired to develop a particle analyzer in which the deterioration of the measurement function is suppressed during the measurement of the particles.

The present invention has been made in view of the above-mentioned prior art, and an object of the present invention is to provide both a method for manufacturing a particle analyzer and the particle analyzer in which the deterioration of the measurement function is suppressed during the measurement of the particles to be measured.

### Means for Solving the Problem

According to the present invention, there is provided a method for manufacturing a particle analyzer and the particle analyzer, as described below.
[1] A method for manufacturing a particle analyzer, the particle analyzer including:
   a first storage chamber in which a first liquid is stored;
   a second storage chamber in which a second liquid containing particles to be analyzed is stored; and
   a flow path connecting the first storage chamber in fluid communication with the second storage chamber,
   the method comprising a surface modification step of irradiating a surface constituting the flow path with an ultraviolet ray to modify the surface of the flow path.
[2] The method for manufacturing a particle analyzer according to [1], wherein the ultraviolet ray irradiated in the surface modification step is a vacuum ultraviolet ray.
[3] The method for manufacturing a particle analyzer according to [1] or [2], wherein the particle analyzer includes a chip having the flow path and an analyzer body housing the chip, and
   wherein the chip is irradiated with the ultraviolet ray to modify the surface constituting the flow path of the chip in the surface modification step.
[4] A particle analyzer including:
   a first storage chamber in which a first liquid is stored;
   a second storage chamber in which a second liquid containing particles to be analyzed is stored; and
   a flow path connecting the first storage chamber in fluid communication with the second storage chamber,
   wherein when a surface constituting the flow path is surface-analyzed by X-ray photoelectron spectroscopy, a relative abundance ratio of C1s on the surface of an inorganic material is 5% or less.

### Effect of the Invention

According to the method for manufacturing a particle analyzer of the present invention, it is possible to manufacture a particle analyzer in which the measurement function is suppressed from deteriorating during the measurement of the particles to be measured.

The particle analyzer of the present invention provides an effect that the measurement function is suppressed from deteriorating during the measurement of the particles to be measured.

### Brief Description of the Drawings

FIG. 1 is a perspective view schematically showing an embodiment of a particle analyzer manufactured by a manufacturing method of the present invention.
FIG. 2 is a cross-sectional view schematically showing an embodiment of a particle analyzer manufactured by a manufacturing method of the present invention.
FIG. 3 is a graph showing the measurement results of the number of particles in Example 1 and Comparative Example 1.
FIG. 4 is a wide spectrum when the surface constituting the flow path of the particle analyzer manufactured according to Example 1 was surface-analyzed by X-ray photoelectron spectroscopy.
FIG. 5 is a wide spectrum when the surface constituting the flow path of the particle analyzer manufactured according to Comparative Example 1 was surface-analyzed by X-ray photoelectron spectroscopy.
FIG. 6 is a graph showing the contact angle of the test plate before and after the vacuum ultraviolet ray irradiation (before and after the surface modification).
FIG. 7 is a photograph showing the measurement state of the contact angle of the test plate of (a) before the surface modification and (b) after the surface modification.

### Mode for Carrying out the Invention

Embodiments of the present invention will be described below with reference to the drawings. It should be understood that the present invention is not limited to the following embodiments, and that changes, improvements, and the like of the design may be added appropriately based on ordinary knowledge of a skill in the art without departing from the spirit of the present invention.

### (1) Method for manufacturing a particle analyzer:

A method for manufacturing a particle analyzer of the present invention is a method for manufacturing a particle analyzer having a first storage chamber in which a first liquid is stored, a second storage chamber in which a second liquid containing particles to be analyzed is stored, and a flow path connecting the first storage chamber in fluid communication with the second storage chamber, and the method has a surface modification step to modify a surface of the flow path by irradiating the surface constituting the flow path with an ultraviolet ray.

According to the method for manufacturing the particle analyzer of the present invention, it is possible to manufacture a particle analyzer in which the measurement function is suppressed from deteriorating during the measurement of the particles to be measured.

### (1-1) Surface modification step:

In the present invention, an operation of ultraviolet ray irradiation described above is performed in the surface modification step. By performing such an operation, the surface of the flow path can be modified. When the surface of the flow path is modified, organic substances (organic contaminants) adhered to the surface of the flow path can be removed, and further, a hydrophilic functional group can be formed on the surface of the flow path.

By removing the organic contaminants adhered to the surface of the flow path and further forming a hydrophilic functional group on the surface of the flow path, it is possible to improve a flow of the particles to be measured passing through the flow path. Specifically, there is a tendency that the particles to be measured are electrically attracted to a surface side of the flow path by the organic contaminants adhered to the surface of the flow path and by the surface state of the flow path, so that the flow path is blocked by the particles. Therefore, by removing the organic contaminants adhered to the surface of the flow path or forming a hydrophilic functional group on the surface of the flow path, it is suppressed that the particles are electrically attracted to the surface side of the flow path, so that the particles hardly adhere to the flow path. As a result, it is possible to suppress the occurrence of a situation in which the measurement function of the particles is deteriorated.

There is no particular limitation on a method of irradiating an ultraviolet ray to the surface constituting the flow path as long as the surface of the flow path can be modified as described above. For example, a method in which an ultraviolet laser is irradiated, a method in which a vacuum ultraviolet laser is irradiated, or the like, using a mixed gas of a rare gas, a halogen, or the like as a laser medium, can be given.

The irradiation time, the irradiation output, and the irradiation distance of the ultraviolet ray can be appropriately determined as long as the surface of the flow path can be modified as described above, and for example, the irradiation time of the ultraviolet ray can be set to 5 to 600 seconds. The illuminance of the ultraviolet ray can be set to 10 to 100W/cm². The irradiation distance can be set to 0.1 to 20mm.

The ultraviolet ray irradiated in the surface modification step is preferably a vacuum ultraviolet ray. The vacuum ultraviolet ray is specifically an ultraviolet ray having a wavelength of 10 to 200nm. By irradiating the vacuum ultraviolet ray, the surface of the flow path can be satisfactorily modified and can be easily modified as compared with a surface treatment by a treatment solution or a surface treatment by oxygen plasma. Specifically, it is possible to satisfactorily remove an organic substance (an organic contaminant) adhered to the surface of the flow path, and further, it is possible to form a large number of hydrophilic functional groups on the surface of the flow path. As a result, it is possible to manufacture a particle analyzer in which the measurement function is greatly suppressed from deteriorating during the measurement of the particles to be measured. By the ultraviolet irradiation, preferably the vacuum ultraviolet irradiation, a relative abundance ratio of C1s on the surface of an inorganic material can be 5% or less when the surface constituting the flow path is surface-analyzed by X-ray photoelectron spectroscopy.

In the surface modification step, at least the surface of the flow path may be modified, and in addition to the surface of the flow path, the surface in the first storage chamber and the surface in the second storage chamber may also be modified. In this way, it is possible to manufacture a particle analyzer in which the measurement function is further suppressed from deteriorating during the measurement of the particles to be measured.

When the particle analyzer includes a chip having a flow path and an analyzer body housing the chip, the surface modification step can be performed as follows. That is, in the surface modification step, the chip is irradiated with the ultraviolet ray to modify the surface constituting the flow path of the chip. In this way, it is possible to certainly modify the surface of the flow path where the particles are easily clogged, and to suppress the deterioration of the measurement function during the measurement of the particles.

The material of the chip is not particularly limited, and the material in which the material itself may be an electrically and chemically inert and insulating may be used. Specific examples of the material of the chip include glass, sapphire, ceramics, resins, rubbers, elastomers, SiO₂, SiN, Al₂O₃, and the like.

Examples of the analyzer body include a laminated body in which plate-like members are laminated (specifically, the plate-like members are sequentially laminated in order from below) as will be described later, those comprising a pair of electrodes, and the like.

The particle analyzer in the present invention is a device for analyzing particles in the following manner. That is, a first liquid stored in a first storage chamber and a second liquid stored in a second storage chamber (the second liquid contains particles to be analyzed such as exosomes) are energized by flowing an electric current, and a current value generated by the energization is measured. At this time, the particles contained in the second liquid in the second storage chamber move to the first storage chamber through the flow path by diffusion of fluid, electrophoresis, or the like. As the particles pass through the flow path, the measured current value changes (increases or decreases) depending on the characteristics (type, shape, size, etc.) of the particles. Therefore, by measuring the current value, it is possible to analyze the characteristics of the particles contained in the second liquid. The particles to be analyzed are not particularly limited, and examples thereof include exosomes, pollens, viruses, bacteria, and DNA.

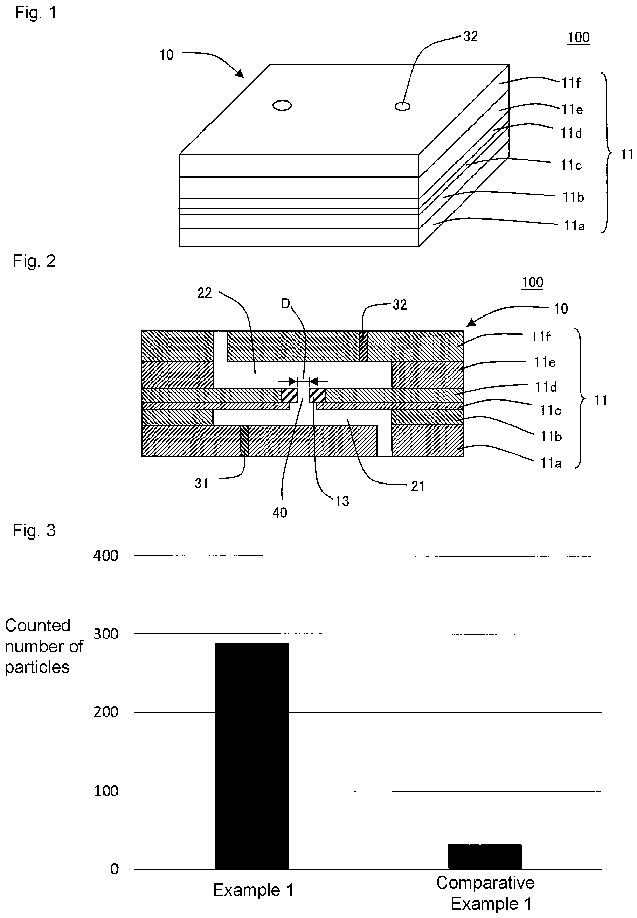

The particle analyzer may include, for example, a particle analyzer 100 as shown in FIGS. 1 and 2. The particle analyzer 100 includes a laminated body 10 in which plate-like members 11 are laminated (specifically, the plate-like members 11a, 11b, 11c, 11d, 11e, and 1 If are laminated in order from below). The laminated body 10 has a first storage chamber 21 in which a first liquid is stored, a second storage chamber 22 in which a second liquid containing particles to be analyzed is stored, and a flow path 40 connecting the first storage chamber 21 in fluid communication with the second storage chamber 22. The particle analyzer 100 further includes a pair of electrodes comprising a first electrode 31 disposed on the first storage chamber 21 and a second electrode 32 disposed on the second storage chamber 22. The laminated body 10 includes a chip 13 having a nanopore (a through-hole in which the diameter is nano-sized) as a flow path 40 connecting the first storage chamber 21 in fluid communication with the second storage chamber 22, at its center. The "plate-like member" of the particle analyzer in the present invention is not limited to a member having a thickness like a plate, and includes a member having a thin thickness, that is, a member having a film shape. The particle analyzer may not include a pair of electrodes.

The material of the plate-like member 11 is not particularly limited as long as it is a material in which the material itself is an electrically and chemically inert and insulating. Examples of the material of the plate-like member 11 include glass, sapphire, ceramics, resins (e.g., acrylic resins), rubbers (e.g., silicone rubbers), elastomers, SiO₂, SiN, and Al₂O₃, and the like.

As described above, the chip 13 may specifically include those made of glass, sapphire, ceramics, resins, rubbers, elastomers, SiO₂, SiN, Al₂O₃, and the like.

In FIGS. 1 and 2, the laminated body 10 employs a configuration in which the chip 13 is provided as a separate body in addition to the plate-like member 11, but in the particle analyzer of the present invention, the plate-like member 11 and the chip 13 may be integrated with each other. Specifically, the laminated body may be provided with a substrate (a plate-like member), a film forming substrate disposed on the substrate and composed of the plate-like member (a film-like body) in which a nanopore (a through-hole in which the diameter is nano-sized) is formed as the flow path 40, and plate-like members disposed on both sides of the film forming substrate. Predetermined grooves are formed in the plate-like members disposed on both sides of the film forming substrate, and the grooves serve as the first storage chamber and the second storage chamber.

The method for producing the laminated body 10 is not particularly limited. For example, it may be a producing method of sequentially laminating plate-like members 11 in which a predetermined groove and the like are already formed, or a producing method employing a photolithography method, an electron beam drawing method, and the like.

### (2) Particle analyzer of the present invention:

The particle analyzer of the present invention can be manufactured by the method for manufacturing a particle analyzer of the present invention described above.

One embodiment of the particle analyzer of the present invention is a particle analyzer 100 shown in FIGS. 1 and 2. The particle analyzer 100 has a first storage chamber 21 in which a first liquid is stored, a second storage chamber 22 in which a second liquid containing particles to be analyzed is stored, and a flow path 40 connecting the first storage chamber 21 in fluid communication with the second storage chamber 22. In the particle analyzer 100, when the surface constituting the flow path 40 (made of an inorganic material) is surface-analyzed by X-ray photoelectron spectroscopy, a relative abundance ratio of C Is on the surface of the inorganic material is 5% or less.

According to the particle analyzer, the measurement function is suppressed from deteriorating during the measurement of the particles to be measured.

C1s is a peak at which the Binding Energy (eV) is observed between 284eV and 292eV among the peaks detected by surface analysis (wide-scan analysis) on the surface constituting the flow path (nanopore) of the particle analyzer by X-ray photoelectron spectroscopy.

In the particle analyzer 100, a relative abundance ratio of C1s on the surface of an inorganic material is 5% or less, preferably 2.5% or less. When the relative abundance ratio of C1s on the surface of the inorganic material is more than 5%, the organic contaminant is not sufficiently removed, and there is a concern that it is difficult to sufficiently suppress the measurement function from deteriorating during the measurement of the particles to be measured.

Here, the "relative abundance ratio of C1s" is a value (%) obtained by calculating from the ratio of an area intensity of a photoelectron intensity (c/s) of C1s with respect to the sum of area intensities of photoelectron intensities (c/s) of detected elements in the wide spectrum obtained by the surface analysis by X-ray photoelectron spectroscopy. That is, the value is calculated by the formula: (the area intensity of the photoelectron intensity (c/s) of C1s / the sum of area intensities of photoelectron intensities (c/s) of the detected element) × 100.

The contact angle after the test plate made of the same inorganic material as the inorganic material constituting the flow path 40 is irradiated with an ultraviolet ray (preferably, a vacuum ultraviolet ray) described above to surface-modify the surface thereof is preferably 20 to 1°, more preferably 5 to 1°.

### Examples

Hereinafter, the present invention will be specifically described based on examples, but the present invention is not limited to these examples.

### (Example 1)

An excimer lamp (EX-mini, manufactured by Hamamatsu Photonics K.K.) was used to irradiate a chip having nanopores and made of Si/SiN with a vacuum ultraviolet ray having a wavelength of 172nm under the condition of the irradiation distance of 1mm and the irradiation times of 15 seconds, thereby surface-modifying the surfaces of the nanopores (flow paths) of the chip. Thereafter, the chip was immediately housed in an analyzer body made of silicone rubber to manufacture a particle analyzer. In the analyzer body, a pair of electrodes is disposed at a predetermined position.

### (Comparative Example 1)

A particle analyzer was manufactured in the same manner as in Example 1 except that the surfaces of the nanopores (flow paths) of the chip were not subjected to the modification treatment.

### (1) Measurement of particle number:

The manufactured particle analyzers (the particle analyzers of Example 1 and Comparative Example 1) were subjected to the particle analysis in the following manner, and the number of particles at that time was measured.

First, a phosphate buffer solution diluted 1 time was injected into a first storage chamber, a second storage chamber, and a flow path of the particle analyzer, and it was confirmed that the first storage chamber and the second storage chamber were in fluid communication through the nanopores. Thereafter, a phosphate buffer solution obtained by mixing standard particles (carboxy group-modified polystyrene having an average particle diameter of 200nm, Polybead Carboxylate, manufactured by Polysciences, Inc.) with a concentration of 0.1% by volume was filled in the second storage chamber, and electrophoresis was generated by applying a voltage of 100mV, and the above standard particles passing through the nanopores were detected. Measurements were performed for 15 minutes, and the number of particles detected (the counted number of particles) at that time was measured. The measurement results of the counted number of particles (N=3) are shown in FIG. 3.

As shown in FIG. 3, the number of particles measured in the particle analyzer of Example 1 was 288, and the number of particles measured in the particle analyzer of Comparative Example 1 was 31.

### (2) Surface analysis:

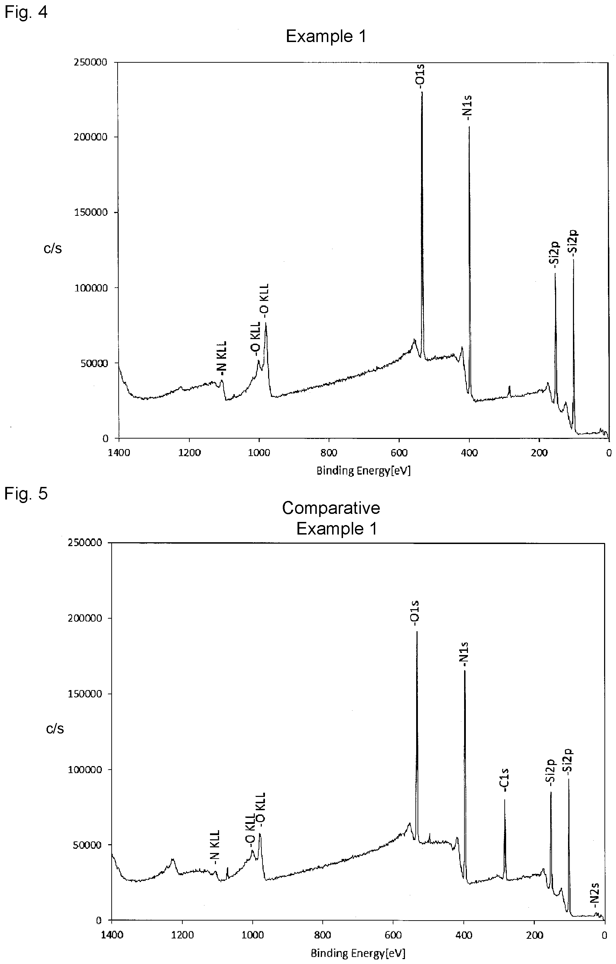

The surface constituting the flow path of the obtained particle analyzer was surface-analyzed by X-ray photoelectron spectroscopy. The results of the surface analysis for Example 1 are shown in FIG. 4, and the results of the surface analysis for Comparative Example 1 are shown in FIG. 5. The relative abundance ratio of each element is shown in Table 1.

As can be seen from FIGS. 4 and 5, in the particle analyzer of Example 1, the peak of the C1s components (organic substances; also referred to as organic contaminants) at the surface constituting the flow path was reduced as compared with the particle analyzer of Comparative Example 1. As can be seen from Table 1, the relative abundance ratio of the C1s components decreased from 19.03% to 2.14%. It is considered that this indicates that the organic contaminants were decomposed and removed by irradiation with the vacuum ultraviolet ray.

**[Table 1]**

| (%) | C1s | N1s | Ols | Si2p |
|---|---|---|---|---|
| Comparative Example 1 | 19.03 | 28.69 | 22.97 | 29.31 |
| Example 1 | 2.14 | 37.91 | 25.76 | 34.2 |

### (3) Measurement of Contact angle:

As a model system, two test plates (25mm in length and 25mm in width) made of SiN were prepared, and the contact angles (contact angles before the surface modification) of the surfaces of these test plates were measured. After that, the surface of the test plate was irradiated with the vacuum ultraviolet ray having a wavelength of 172nm under the condition of the irradiation distance of 1mm and the irradiation times of 15 seconds which is the same irradiation condition as the particle analyzer manufacturing condition, thereby surface-modifying the surface of the test plate. The irradiation condition is the same irradiation condition as the particle analyzer manufacturing condition. Thereafter, the contact angle on the surface of the test plate after the surface modification was measured. Specific measurement conditions are shown in Table 2. Before and after the surface modification, the measuring parts (10 points) of the contact angle is the same position.

**[Table 2]**

| Measurement conditions | |
|---|---|
| Measurement method | Sessile drop method (1µL of pure water) |
| Measuring device | DropMaster500 (Kyowa Interface Science Co., Ltd.) |
| Measuring point | 10 points (5 points/25mm square SiN×2 plates) |

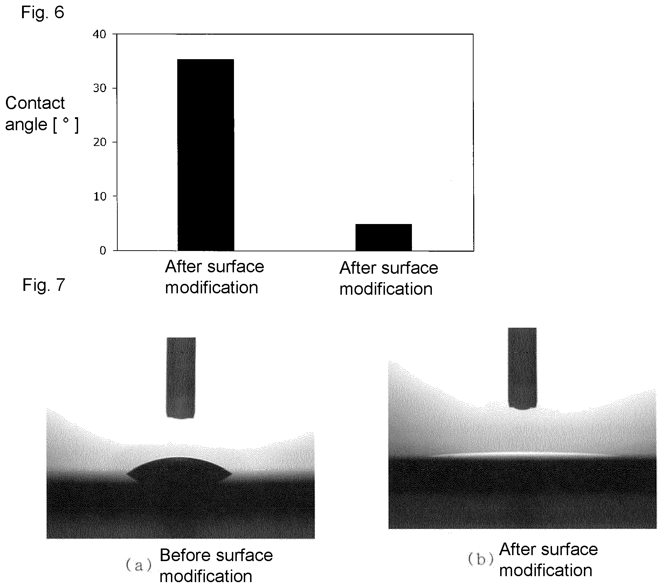

As a result of the measurement of the contact angle, the contact angle of the test plate decreased from 35° to 5° (see FIG. 6). From the result, it is considered that, even in the particle analyzer of Example 1, the surface constituting the flow path is hydrophilized (that is, a hydrophilic functional group is formed) by irradiation with the vacuum ultraviolet ray. The contact angle was measured at 10 points (see Table 2) and the average value was calculated. In Table 2, the "25mm square SiN" means that the square test plate (25mm in length and 25mm in width) made of SiN each was a test part. The measuring parts were the four corners and the center part of each test plate (5 points in total).

FIG. 7 shows the measurement state of the contact angle of the test plate before and after the surface modification, (a) shows the measurement state of the test plate before the surface modification, and (b) shows the measurement state of the test plate after the surface modification. The results of FIG. 7 also show that the contact angle decreases before and after the surface modification.

From the results of Example 1 and Comparative Example 1, it was confirmed that the particle analyzer manufactured by the method for manufacturing the particle analyzer of Example 1 was suppressed from deteriorating the measurement function during the measurement of the particles to be measured, compared to the particle analyzer manufactured by the method for manufacturing the particle analyzer of Comparative Example 1.

### Industrial Applicability

The method for manufacturing the particle analyzer of the present invention can be adopted as a method for manufacturing a particle analyzer for analyzing particles such as exosomes, pollen, viruses, bacteria, and the like. In addition, the particle analyzer of the present invention can be adopted as a particle analyzer for analyzing particles such as exosomes, pollen, viruses, and bacteria.

### Description of Reference Numerals

10: laminated body, 11: plate-like member, 13: chip, 21: first storage chamber, 22: second storage chamber, 31: first electrode, 32: second electrode, 40: flow path, 100: particle analyzer.

## Claims

1. A method for manufacturing a particle analyzer, the particle analyzer comprising:
a first storage chamber in which a first liquid is stored;
a second storage chamber in which a second liquid containing particles to be analyzed is stored; and
a flow path connecting the first storage chamber in fluid communication with the second storage chamber,
the method comprising a surface modification step of irradiating a surface constituting the flow path with an ultraviolet ray to modify the surface of the flow path.

2. The method for manufacturing a particle analyzer according to claim 1, wherein the ultraviolet ray irradiated in the surface modification step is a vacuum ultraviolet ray.

3. The method for manufacturing a particle analyzer according to claim 1 or 2, wherein the particle analyzer includes a chip having the flow path and an analyzer body housing the chip, and
wherein the chip is irradiated with the ultraviolet ray to modify the surface constituting the flow path of the chip in the surface modification step.

4. A particle analyzer comprising:
a first storage chamber in which a first liquid is stored;
a second storage chamber in which a second liquid containing particles to be analyzed is stored; and
a flow path connecting the first storage chamber in fluid communication with the second storage chamber,
wherein when a surface constituting the flow path is surface-analyzed by X-ray photoelectron spectroscopy, a relative abundance ratio of C1s on the surface of an inorganic material is 5% or less.
